Europäisches Patentamt

European Patent Office

Office européen des brevets

(11) Publication number: **0 160 240 B1**

# EUROPEAN PATENT SPECIFICATION

(45) Date of publication of patent specification: **31.07.91**

(51) Int. Cl.⁵: **G01N 33/84**, C12Q 1/12, G01N 33/52

(21) Application number: **85104305.9**

(22) Date of filing: **10.04.85**

(54) Test piece for detecting nitrite.

(30) Priority: **02.05.84 JP 87858/84**

(43) Date of publication of application:
**06.11.85 Bulletin 85/45**

(45) Publication of the grant of the patent:
**31.07.91 Bulletin 91/31**

(84) Designated Contracting States:
**BE DE FR IT**

(56) References cited:
**EP-A- 0 016 962      EP-A- 0 037 056**
**EP-A- 0 043 469      EP-A- 0 103 958**
**DE-A- 2 011 679      US-A- 3 411 887**
**US-A- 3 712 853**

(73) Proprietor: **TERUMO KABUSHIKI KAISHA trading as TERUMO CORPORATION**
**44-1, 2-chome, Hatagaya Shibuya-Ku**
**Tokyo 151(JP)**

(72) Inventor: **Kaminagayoshi, Satoshi**
**24-19, Senbonkita 2-chome Nishinari-ku**
**Osaka-shi Osaka(JP)**

(74) Representative: **Henkel, Feiler, Hänzel & Partner**
**Möhlstrasse 37**
**W-8000 München 80(DE)**

## Description

Field of the Invention:

This invention relates to a test piece which is not susceptible to the influence of a reducing substance, and particularly to an improved test piece for detecting a nitrite of microorganic origin in urine, in a test piece for detecting a nitrite by utilizing the diazo coupling reaction.

Description of Prior Art:

Recently, rapid diagnostic agents are gaining notably in significance in medical practice and in clinical tests. Rapid diagnostic agents having absorbent carriers as their bases are important. When an absorbent carrier which, in most cases, is paper is impregnated with a chemical agent necessary for the reaction of detection and immersed in a given body fluid specimen, it undergoes a color reaction if the substance under detection is present in the specimen. A rapid diagnostic agent for the detection of a nitrite is one example of the rapid diagnostic agents which are now gaining greatly in significance in medical diagonosis. Nitrite is produced by the nitrate-reducing activity of a specific bacterium such as, for example, Escherichia coli, para Escherichia coli, Salmonella typhi, Salmonella paratyphi, staphytococcus or enterococcus. The detection of a nitrite, particularly a nitrite in urine is important for the diagnosis of infection with a bacterium or other microorganism which constitutes a pathogen for the human body.

The test under discussion is based on the following principle. In the detection of a nitrite, the nitrite is caused in an acidic condition to react with a diazotizing agent to produce a diazo compound and this diazo compound is reacted upon by a coupling agent to give rise to an azo compound possessing red chromophore, azo group -N = N-. The color of the azo compound serves as a criterion for the amount of the nitrite present because the density of this color has a sufficient correlation with the concentration of nitrite ions.

Recent processed foodstuffs and refreshing beverages contain ascorbic acid in large amount as vitamin C and generous ingestion of vitamin preparations for the preservation of health is in vogue. Thus, such vitamins are richly contained in blood and excess water-soluble vitamins are discharged out of the human body as entrained in urine. When the aforementioned diagnostic agent is used in the test of urine or blood, therefore, there is entailed the problem that the strong reducing power exhibited by the ascorbic acid present in the urine or blood will interfere with the diazo coupling reaction and impede ample coloration.

EP-A-0 037 056 discloses a test strip for the detection of bilirubin in body fluids. The disturbing effect of any reducing agents, mainly ascorbic acid, is eliminated by addition of a soluble iodate compound.

US-A-3 411 887 discloses a test composition for detecting specific constituents in body fluids, which contain interfering reducing substances such as ascorbic acid. The known test composition comprises an enzymatic detecting system which releases peroxide and a trapping system for oxydizing the interfering reducing substance. Said trapping system comprises an ionizable heavy metal compound.

EP-A-0 103 958 discloses a solid support "dip-stick" immunoassay wherein a peroxidase catalyses the formation of a dye. Ascorbate, which interferes with the assay is reduced or eliminated by impregnating the support with periodate.

DE-A-20 11 679 and US-A-3 712 853 disclose test-strips for checking nitrate by virtue of the diazo coupling reaction. According to said documents nitrates, e.g. nitrates formed by bacteria in body fluids, are detected even in very small concentrations with a diagnostic test reaction comprising a diazoticable amine, a solid organic acid, and a coupling component. The problem of strong reducing agents present in the urine or blood are not disclosed in said references, nor is an oxidizing agent incorporated in said diagnostic test reactions.

An object of this invention, therefore, is to provide a novel test piece which effects detection of a nitrite by virtue of the diazo coupling reaction without being affected by any reducing substance.

Another object of this invention is to provide a test piece which excels in stability appropriate for the detection of a nitrite of microorganic origin present in urine.

## SUMMARY OF THE INVENTION

The objects described above are attained by a test piece for detecting a nitrite by virtue of the diazo coupling reaction, comprising on a substrate a diazotizing agent and a coupling agent for the diazo reaction, $NaIO_4$ in an amount of 0.9 to 15 mmol/m$^2$ of said test piece and a substance to produce an acidic condition.

DESCRIPTION OF THE PREFERRED EMBODIMENT

In the test, the aforementioned oxidizing agent is used in an amount large enough to give effective prevention of the inhibition of the reaction of an oxidizing indicator by reducing substances such as ascorbic acid present in the specimen under test. The effective amount of the oxidizing agent is 0.9 to 15 mmol/m$^2$ in terms of equivalent weight.

The diazotizing agent to be contained in the test piece of the present invention is intended to react in an acidic condition with a nitrite contained in the specimens under test to form a diazo compound. In the aromatic primary amines, arsanilic acid and sulfanylamide are desirably used.

Examples of the coupling agent to be contained in the test piece according to this invention include α-naphthylamines, N-substituted-α-naphthylamines, N-(1-naphthyl)-ethylenediamine, and N-alkyl-α-naphthylamines and N,N-di-substituted-α-naphthylamines such as, N,N-dialkyl- -naphthylamines and salts thereof. Among the examples cited above, N-(1-naphthyl)-ethylenediamine dihydrochlorides and N,N-dimethyl-α-naphthylamine prove particularly desirable.

Examples of the substance which is used to produce the acidic condition in the reaction of the diazotizing agent with the nitrite during the detection of the nitrite by the test piece of this invention include tartaric acid, citric acid, oxalic acid, malic acid, malonic acid, succinic acid, glutaric acid and adipic acid. This substance is contained in corporation with the aforementioned chemical agents in the test piece.

A wetting agent is used enable the test piece immersed in the specimen to be uniformly wetted with the specimen. Examples of the wetting agent include surface active agents represented by alkyl sulfates such as sodium laurylsulfate, sodium dodecylsulfate and sodium tetradodecylsulfate, alkylbenzenesulfonates such as sodium dodecylbenzenesulfonate, and sodium diheptylsulfosuccinate.

The test piece obtained by impregnating the substrate with chemical agents may use therein a viscous agent adapted to prevent the contained chemical agents from exuding. Typical examples of the viscous agent include polymers such as polyvinyl alcohol, polyvinyl pyrrolidone, polyethylene glycol, polyacrylates, polyacrylamides, poly(hydroxyethyl methacrylate), poly(hydroxyethyl acrylate), carboxymethyl cellulose, and gelatin and gum arabic.

The use of the aforementioned chemical agents in the test piece of this invention is accomplished by dissolving these chemical agents in a solvent such as, for example, benzene, toluene, xylene, methanol, ethanol, isopropanol, acetone, methylethyl ketone, chloroform, carbon tetrachloride or water, impregnating the substrate with the resultant solution, and drying the substrate wet with the chemical agents.

Examples of the substrate include filter paper and other similar forms of paper, non-woven fabrics formed of glass fibers or plastic material, and water-absorbing plastic sheets. Any of these substrates may be effectively used on the condition that it is incapable of reacting with the impregnating solution, insoluble in the solution, and capable of absorbing the solution. Paper is a preferred example.

After the test piece is produced by being impregnated with the solution of the chemical agents and subsequently dried, a thin peelable film of polyethylene, polyvinyl chloride, polyvinylidene chloride, or nylon may be applied on the test piece optionally for the purpose of protecting the test piece against deterioration of quality by humidity or ambient gas during the storage or service of the test piece and protecting the test piece against damage. This protective film is separated from the test piece immediately before the test piece is put to use.

Now, the present invention will be described more specifically below with reference to working examples.

## Example 1

| Solution A: | Arsanilic acid | 0.8 g |
| | Methanol | 100 ml |
| Solution B: | N-(1-naphthyl) ethylenediamine dihydrochloric acid salt | 0.9 g |
| | Methanol | 100 ml |
| | Polyvinyl pyrrolidone | 10.0 g |
| Solution C: | Oxidizing agent (NaIO$_4$) | 100 mg |
| | Distilled water | 100 ml |

A filter paper was impregnated with Solution A and dried. It was then impregnated with Solution B and dried. Finally, the filter paper was impregnated with Solution C and dried. Consequently, there was obtained a test piece for a nitrite. In specimens prepared by incorporating 50 mg/dl of ascorbic acid respectively in urine containing no $NaNO_2$ and urine containing $NaNO_2$ in a concentration of 1 mg/dl, samples of the test piece obtained as described above were immersed for test. The results were as shown in Table 1.

Control 1

The procedure of Example 1 was repeated, except that only Solution A and Solution B were used in the preparation of the test piece. The results are shown in Table 1.

### Table 1

| | Oxidizing agent | Urine containing ascorbic acid in a concentration of 50 mg/dl and no $NaNO_2$ | Urine containing ascorbic acid in a concentration of 50 mg/dl and $NaNO_2$ in a concentration of 0.1 mg/dl |
|---|---|---|---|
| Example 1 | $NaIO_4$ | − | + |
| Control 1 | − | − | − |

Note: + Coloration observed.
− Absence of coloration.

Example 2

| | | |
|---|---|---|
| Solution A: | Sulfanylamide | 104 mg |
| | Methanol | 20 ml |
| Solution B: | Tartaric acid | 0.5 g |
| | Polyvinyl pyrrolidone, K30 | 2 g |
| | $NaIO_4$ | 20 mg |
| | Water | 20 mg |
| Solution C: | Dimethyl naphthaylamine | 692 mg |
| | Benzene | 20.2 mg |

A test piece was obtained by using the aforementioned solutions, Solution A, Solution B and Solution C, and following the procedure of Example 1. The results were as shown in Table 2.

Control 2

The procedure of Example 2 was repeated, except that $NaIO_4$ was omitted from Solution B. The test piece was tested by following the procedure of Example 1. The results were as shown in Table 2.

Table 2

| | Ascorbic acid (mg/dl) | Reaction of Example 2 | Reaction of Control 2 |
|---|---|---|---|
| Urine containing no NaNO$_2$ | 0 | – | – |
| Urine containing NaNO$_2$ in a concentration of 0.1 mg/dl | 0 | ++ | ++ |
| " | 25 | ++ | – |
| " | 50 | ++ + | – |
| " | 100 | + | – |

Note: ++ Strong coloration observed.

+ Coloration observed.

– Absence of coloration.

As described above, this invention relates to a test piece used for detection and measurement of a nitrite by virtue of the diazo coupling reaction. Even when a given specimen such as urine happens to contain reducing substances such as ascorbic acid, the diazo coupling reaction the test piece produces in the specimen is not impeded because the reducing substances are oxidized by the aforementioned oxidizing agent. The measurement of a nitrite by the test piece of this invention is excellent when arsanilic acid or sulfanylamine is used as the diazotizing agent and N-(1-naphthyl)-ethylenediamine hydrochlorides or N,N-dimethyl-naphthylamine is used as the coupling agent.

**Claims**

1. A test piece for detecting a nitrite by virtue of the diazo coupling reaction, comprising on a substrate a diazotizing agent and a coupling agent for the the diazo reaction, NaIO$_4$ in an amount of 0.9 to 15 mmol/m$^2$ of said test piece and a substance to produce an acidic condition.

2. A test piece according to Claim 1, wherein said diazotizing agent is an aromatic primary amine.

3. A test piece according to Claim 1, wherein said diazotizing agent is arsanilic acid or sulfanylamide.

4. A test piece according to any of Claims 1 - 3, wherein said coupling agent is selected from the group consisting of α-naphthylamines, N-substituted-α-naphthylamines,N,N-disubstituted-α-naphthylamines and N-(1-naphthyl)ethylenediamine.

5. A test piece according to Claim 4, wherein said coupling agent is N,N-dimethylnaphthylamine or N-(1-naphthyl)-ethylenediaminehydrochlorides.

6. A test piece according to any of Claims 1 to 5, wherein said substrate is selected from the group consisting of paper, non-woven fabric of glass fibers or plastic material and water-absorbing plastic sheet.

7. A test piece according to Claim 6, wherein said substrate is paper.

**Revendications**

1. Une pièce d'essai pour détecter un nitrite par la réaction de couplage diazo, comprenant sur un substrat un agent de diazotation et un agent de couplage pour la réaction diazo, du NaIO$_4$ en quantité de 0,9 à 1 mmol/m$^2$ de ladite pièce d'essai et une substance pour produire des conditions acides.

2. Une pièce d'essai selon la revendication 1, selon laquelle ledit agent de diazotation est une amine primaire aromatique.

3. Une pièce d'essai selon la revendication 1, selon laquelle ledit agent de diazotation est l'acide arsanilique ou le sulfanylamide.

4. Une pièce d'essai selon l'une quelconque des revendications 1 à 3, selon laquelle ledit agent de couplage est choisi dans le groupe suivant α-naphtylamines, α-naphtylamines N-substituées, α-naphtylamines N,N-disubstituées et N-(1-naphtyl)-éthylènediamine.

5. Une pièce d'essai selon la revendication 4, selon laquelle ledit agent de couplage est la N,N-iméthylnaphtylamine ou les chlorhydrates de N-(1-naphtyl)-éthylènediamine.

6. Une pièce d'essai selon l'une quelconque des revendications 1 à 5, selon laquelle ledit substrat est choisi dans le groupe comprenant le papier, les tissus non tissés en fibres de verre ou en matières plastiques et les feuilles plastiques absorbant l'eau.

7. Une pièce d'essai selon la revendication 6, selon laquelle ledit substrat est du papier.

**Patentansprüche**

1. Testbesteck zum Nachweis eines Nitrits mit Hilfe der Diazokupplungsreaktion, enthaltend auf einem Substrat ein Diazotierungsmittel und ein Kupplungsmittel für die Diazoreaktion, NaIO$_4$ in einer Menge von 0,9 - 15 mMol/m$^2$ des Testbestecks und eine Substanz zur Herbeiführung eines sauren Zustands.

2. Testbesteck nach Anspruch 1, dadurch gekennzeichnet, daß das Diazotierungsmittel aus einem aromatischen primären Amin besteht.

3. Testbesteck nach Anspruch 1, dadurch gekennzeichnet, daß das Diazotierungsmittel aus Arsanilsäure oder Sulfanylamid besteht.

4. Testbesteck nach einem der Ansprüche 1 bis 3, dadurch gekennzeichnet, daß das Kupplungsmittel aus der Gruppe α-Naphthylamine, N-substituierte α-Naphthylamine, N,N-disubstituierte α-Naphthylamine und N-(1-Naphthyl)-ethylendiamin ausgewählt ist.

5. Testbesteck nach Anspruch 4, dadurch gekennzeichnet, daß das Kupplungsmittel aus N,N-Dimethylnaphthylamin oder N-(1-Naphthyl)-ethylendiamin-hydrochloriden besteht.

6. Testbesteck nach einem der Ansprüche 1 bis 5, dadurch gekennzeichnet, daß das Substrat aus der Gruppe Papier, Gespinst oder Gewirk aus Glasfasern oder Kunststoffmaterial und einer wasserabsorbierenden Kunststoffolie ausgewählt ist.

7. Testbesteck nach Anspruch 6, dadurch gekennzeichnet, daß das Substrat aus Papier besteht.